# EUROPÄISCHE PATENTANMELDUNG

(11) **EP 2 886 121 A1**
(43) Veröffentlichungstag der Anmeldung: **24.06.2015**
(21) Anmeldenummer: 13199321.4
(22) Anmeldetag: 23.12.2013
(51) Int. Cl.: A61K 31/58, A61K 9/10

(54) **Wässrige Suspension enthaltend Budesonid zur Behandlung von entzündlichen Veränderungen des Ösophagus**

(71) Anmelder: Dr. Falk Pharma GmbH, 79108 Freiburg (DE)
(72) Erfinder: Greinwald, Roland, 79341 Kenzingen (DE); Müller, Ralph, 79111 Freiburg (DE); Wilhelm, Rudolf, 76476 Bischweier (DE)
(74) Vertreter: Keller, Günter

(57) **Zusammenfassung**

Offenbart wird eine wässrige Suspension enthaltend Budesonid, die einen pH-Wert von 4,0 bis 5, 0 aufweist und vor allem in der Pädiatrie Verwendung findet.

## Beschreibung

Für die Behandlung von entzündlichen Prozessen und Veränderungen der Speiseröhre, wie zum Beispiel der eosinophilen Ösophagitis, ist eine Arzneiform erforderlich, die nach oraler Anwendung die lokale Verfügbarkeit des Wirkstoffes Budesonid in genügend hoher Konzentration am Entzündungsort ermöglicht. Dieses als Ösophagus- bzw. SpeiseröhrenTargeting bezeichnete Konzept ist mit der oralen Applikation einer einfachen Wirkstofflösung nicht realisierbar, da ein hohes Risiko besteht, dass der Wirkstoff dann rasch und annährend quantitativ in den Magen geschluckt wird. Das Ziel des Ösophagus- bzw. Speiseröhrentargeting soll daher bevorzugt dadurch erreicht werden, dass ein langsames Entlanggleiten des Wirkstoffes an der Schleimhaut der Speiseröhre verbunden mit einer vollständigen Benetzung der Oberfläche sowie einer Anhaftung des Wirkstoffes ermöglicht wird. Diese Art der Anwendung bringt den Wirkstoff gezielt an den Wirkort. Weiterhin ist zu berücksichtigen, dass die therapeutische Behandlung der Speiseröhre idealerweise noch zusätzliche spezielle Anforderungen an die Arzneiform erfordert, je nachdem welche Patientenpopulation behandelt werden soll. Die vorliegende Erfindung ist speziell geeignet, eine altersgerechte Arzneiform zur Verfügung zu stellen, die bei Kindern einfach und verlässlich anzuwenden ist und somit eine hohe Einhaltungsquote der verschriebenen Tagesdosis ("Compliance") erreicht.

Es gibt verschiedene Formen von entzündlichen Erkrankungen der Speiseröhre, die mit der erfindungsgemäßen Suspension behandelt werden können. Die eosinophile Ösophagitis ist eine chronische, inflammatorische Erkrankung der Speiseröhre, die mit einer Funktionseinschränkung des Ösophagus (Speiseröhre) einhergeht und durch eine Infiltration des Speiseröhrenepithels mit eosinophilen Granulozyten gekennzeichnet ist. Seit Ende der '70er Jahre wurde die eosinophile Ösophagitis kasuistisch beschrieben und seit Ende der '90er Jahre vermehrt diagnostiziert. Es scheint eine durch Th2-Zellen vermittelte Antwort auf aerogene und auf durch Nahrungsmittel aufgenommene Allergene vorzuliegen, die zur Ausschüttung von IL-13, IL-5 und nachfolgend zur vermehrten Produktion von Eotaxin-3 führt. Hierdurch werden eosinophile Granulozyten angelockt. Klinisch stehen eine häufig lange bestehende Dysphagie, sowie Bolusimpaktationen im Vordergrund. Die Diagnosestellung erfordet standardgemäß den Nachweis von ≥ 15 Eosinophilen / high power field in der Speiseröhre bei Patienten mit Symptomen einer Funktionsstörung des Ösophagus. Bei manchen Patienten sind lediglich leicht zu übersehende Schleimhautveränderungen zu beobachten. Die eosinophile Ösophagitis tritt sowohl im Kindes-, wie auch im jüngeren Erwachsenenalter gehäuft auf.

Die WO 2009/064417 offenbart Zusammensetzungen für die Behandlung von gastrointestinalen Entzündungen. Die dort beschriebenen Zusammensetzungen umfassen ein Corticosteroid und wenigstens ein zusätzliches Mittel zur Behandlung der Entzündung. In der WO 2009/064457 werden Corticosteroid enthaltende Zusammensetzungen offenbart, die zur Behandlung von Entzündungen des Gastrointestinaltrakts geeignet sind.

Die US 2007/0111978 beschreibt Verfahren zur Linderung von entzündlichen Erkrankungen des Gastrointestinaltrakts. Vorgeschlagen wird dort die Herstellung einer hochviskosen Lösung aus einer Budesonid-Suspension mit einer sehr hohen Konzentration an Sucralose. Die US 2009/0264392 beschreibt Verfahren und Zusammensetzungen, die geeignet sind zur Behandlung der eosinophilen Ösophagitis. Bei dieser Behandlungsmethode wird ein Steroid und ein an der Schleimhaut haftendes Mittel eingesetzt.

In der US 2011/0097401 werden Verfahren zur Behandlung von gastrointestinalen Erkrankungen beschrieben. Die US-Patentanmeldung beschreibt insbesondere Mikropellets sowie Brausetabletten und Verfahren zur Linderung von gastrointestinalen Entzündungen.

Es ist eine Aufgabe der vorliegenden Erfindung, pharmazeutische Formulierungen bereitzustellen, die einerseits Vorteile bei der Applikation mit sich bringen und die andererseits gut hergestellt und in einer lagerstabilen Form bereitgestellt werden können. Die erfindungsgemäße wässrige Suspension ist vor allem für die Behandlung von Kindern konzipiert. Um eine geeignete pharmazeutische Formulierung bereitstellen zu können, die von Kindern akzeptiert wird und, die von der Verabreichung so einfach ist, dass keine Fehler auftreten, ist eine wässrige Suspension besonders geeignet. Diese Suspension kann mit Hilfe von geeigneten Meßlöffeln genau dosiert werden. Erforderlich ist, dass derartige wässrige Suspensionen von den Kindern gut angenommen werden. Daher muss die Viskosität genau eingestellt werden, weil einerseits die Suspension von den Kindern angenommen werden muss und daher nicht zu dickflüssig sein darf. Andererseits soll der Wirkstoff nach dem Schlucken an der Schleimhaut der Speiseröhre ausreichend lange haften, damit sich die topische Wirkung des pharmazeutisch aktiven Wirkstoffes entfalten kann.

Aus dem Stand der Technik ist die Art der Anwendung von Brausetabletten bekannt, die nach Auflösung in Wasser als Trink- oder Spüllösung angewendet werden. Diese Anwendung des Wirstoffes hat für die beschriebene Indikation und vor allem bei der Verabreichung an Kinder verschiedene Nachteile, wie beispielsweise die Verwendung eines größeren Volumens an Wasser (üblicherweise 100 ml bis 250 ml) und die damit verbundene Verdünnung des Wirkstoffes bzw. die Verteilung des Wirkstoffes in der gesamten Mundhöhle sowie das üblicherweise praktizierte rasche Hinunterschlucken der Lösung in den Magen. Gerade für die Anwendung bei Kindern ist die Zubereitung von Brausetabletten ungeeignet. Einerseits wird Zeit für die Herstellung der Lösung benötigt, andererseits ist das mit der Brausetablette zubereitete Wirkstoffvolumen der wirkstoffhaltigen Lösung häufig zu groß und die Kinder nehmen nicht die ganze Menge auf.

Bekannt ist auch die Anwendung von Budesonid bei der eosinophilen Ösophagitis (Straumann et al., Gastroenterology, 2010, S. 1526-1537). Allerdings wurden in dieser Studie Flüssigampullen, sogenannte "Respules" verwendet, deren bestimmungsgemäßer Gebrauch die Inhalation ist, wobei der Ampulleninhalt vorab in einen Zerstäuber gefüllt und dann inhaliert wird. Abweichend von diesem bestimmungsgemäßen Gebrauch wurde in der zitierten Studie die Budesonidsuspension der Ampulle als Trinklösung angewendet.

Für die Behandlung von entzündlichen Prozessen und Veränderungen der Speiseröhre, wie zum Beispiel der eosinophilen Ösophagitis, ist eine Arzneiform erforderlich, die nach oraler Anwendung die lokale Verfügbarkeit des Wirkstoffes Budesonid in genügend hoher Konzentration am Entzündungsort ermöglicht. Dieses als Ösophagus- bzw. SpeiseröhrenTargeting bezeichnete Konzept soll daher bevorzugt dadurch erreicht werden, dass ein langsames Entlanggleiten des Wirkstoffes an der Schleimhaut der Speiseröhre verbunden mit einer vollständigen Benetzung der Oberfläche sowie einer Anhaftung des Wirkstoffes ermöglicht wird. Diese Art der Anwendung bringt den Wirkstoff gezielt an den Wirkort. Weiterhin ist zu berücksichtigen, dass die therapeutische Behandlung der Speiseröhre idealerweise noch zusätzliche spezielle Anforderungen an die Arzneiform erfordert, je nachdem welche Patientenpopulation behandelt werden soll. Die vorliegende Erfindung ist speziell geeignet, eine altersgerechte Arzneiform zur Verfügung zu stellen, die bei Kindern einfach, verlässlich und bezüglich der Dosierung flexibel anzuwenden ist und somit eine hohe Einhaltungsquote der verschriebenen Tagesdosis ("Compliance") erreicht. Gleichzeitig muss eine ausreichende Akzeptanz der Darreichungsform in der Zielpopulation sichergestellt werden.

Die vorliegende Erfindung betrifft pharmazeutische Formulierungen, insbesondere eine flüssige Zubereitung zum Einnehmen, die als pharmazeutischen Wirkstoff Budesonid oder ein pharmazeutisch verträgliches Salz oder Derivat davon umfasst und bevorzugt in der Pädiatrie zur Behandlung von entzündlichen Erkrankungen der Speiseröhre angewendet wird.

Es ist daher eine Aufgabe der vorliegenden Erfindung, unter Berücksichtigung der Indikation eine für diese Zielpopulation geeignete, lager- sowie anwendungsstabile und industriell herstellbare Darreichungsform von Budesonid bereitzustellen. Diese Darreichungsform ist üblicherweise eine flüssige Zubereitung. Flüssige Zubereitungen zum Einnehmen für Kinder sind überwiegend wässrige Präparate, die zur Gewährleistung der physikalisch-chemischen und mikrobiologischen Stabilität sowie zur Gewährleistung der therapeutischen Wirksamkeit neben Wasser weitere geeignete Hilfsstoffe enthalten müssen, um die Anforderungen an die pharmazeutische Qualität derartiger Darreichungsformen zu erfüllen. Gleichzeitig müssen die Hilfsstoffe das Anhaften sowie die Verweildauer des arzneilich wirksamen Bestandteils an der Schleimhaut der Speiseröhre begünstigen. Die flüssige Zubereitung kann sowohl in Einzel- als auch in Mehrdosenbehältnissen angeboten werden. Mehrdosenbehältnisse erlauben neben der Mehrfachentnahme in Abhängigkeit von der gewählten Dosiervorrichtung auch die Entnahme unterschiedlicher bzw. individueller Dosierungen. Dies ist für die Anwendung in der Pädiatrie besonders wichtig.

Die erfindungsgemäße flüssige Zubereitung zum Einnehmen ist ein Zwei-Phasen-System in Form einer Suspension. Es handelt sich dabei um ein feindisperses System vom Typ fest-in-flüssig. Dabei ist der arzneilich wirksame Bestandteil Budesonid homogen in einer kohärenten wässrigen Außenphase dispergiert. Nur durch die Kombination von Wirkstoffeigenschaften mit einer besonders zusammengesetzten kohärenten Außenphase sowie der Auswahl eines geeigneten Behältnisses ist die Erfindung speziell dazu geeignet, eine altersgerechte Arzneiform zur Verfügung zu stellen, die bei Kindern einfach, sicher und verlässlich anzuwenden ist.

Dabei wird die Bereitstellung einer stabilen, sicheren und wirksamen Suspension zur pädiatrischen Anwendung von Budesonid bei der Behandlung von entzündlichen Erkrankungen der Speiseröhre dadurch ermöglicht, dass der arzneilich wirksame Bestandteil in der erfindungsgemäßen Partikelgrößenverteilung in der bezüglich des pH-Wertes, der Viskosität, der Dichte, der chemischen, physikalischen und mikrobiologischen Stabilisierung sowie der bioadhäsiven Eigenschaften und des Geschmacks erfindungsgemäß zusammengesetzten und hergestellten wässrigen kohärenten Außenphase formuliert und in einem geeigneten Behältnis angewendet wird. Die vorliegende Erfindung beschreibt die qualitative und quantitative Zusammensetzung der oralen wässrigen Suspension aus arzneilich wirksamem Bestandteil, den erforderlichen Hilfsstoffen sowie den geeigneten Behältnissen für die Einmal- und Mehrfachentnahme der Suspension zur Anwendung bei Kindern.

Die Stabilitätseigenschaften von Budesonid sind allgemein bekannt. Der Wirkstoff ist hydrolyse-, oxidations- und lichtempfindlich, so dass entsprechende Stabilisierungsmaßnahmen besonders in flüssigen und halbfesten Formulierungen erforderlich sind, um eine lagerstabile Zubereitung zu erhalten.

Überraschenderweise konnte nun festgestellt werden, dass durch die Einstellung des pH-Wertes der wässrigen Außenphase auf 4.0 bis 5.0 sowie den Zusatz von bis zu maximal 0,1 Gew.-% des Natriumsalzes der Ethylendiamintetraessigsäure (Na₂EDTA) eine chemische Stabilisierung von Budesonid in der Suspension ermöglicht wird. Für die Einstellung des erforderlichen pH-Wertes eignen sich vor allem organische Säuren, insbesondere aber die Zitronensäure. Bereits der Zusatz von 0,01 bis 0,1 Gew.-% an Citronensäure ist ausreichend, um den pH-Wert einzustellen und während der Lagerung der Zubereitung sowie nach Anbruch des Behältnisses bei Mehrfachentnahme über eine Anwendungsdauer von mindestens 14 Tagen stabil zu halten. Innerhalb dieses pH-Wertbereichs wird der hydrolytische Abbau von Budesonid verhindert. Dabei erfolgt die Bestimmung des pH-Wertes bevorzugt nach der European Pharmacopea gemäß der Monographie 2.2.3.

Da es sich bei der Citronensäure um eine mehrwertige Säure handelt, kann Citronensäure bzw. geeignete Salze hiervon als Puffer eingesetzt werden. Durch Zugabe von Citronensäure bzw. geeigneten Salzen der Citronensäure kann diese Pufferwirkung bequem eingestellt werden.

Der Zusatz von nur geringen Mengen an Na₂EDTA verhindert allein die Autoxidation von Budesonid, indem Spuren von katalytisch wirkenden Metallionen komplexiert werden. Der Zusatz weiterer Antioxidantien ist daher nicht erforderlich. Dies wird auch durch den eingestellten pH-Wert von 4.0 bis 5.0 ermöglicht, der eine ausreichende Komplexbildungsaktivität des Chelatbildners sicherstellt. Die erfindungsgemäße Zusammensetzung der Hilfsstoffe zur pH-Wert-Stabilisierung und zur Antioxidation ist besonders einfach und mit Blick auf die Zielgruppe (Kinder) besonders vorteilhaft. Diese Hilfsstoffe sind unbedenklich und es wird der Zusatz weiterer Stabilisatoren vermieden.

Wässrige flüssige Zubereitungen zur peroralen Anwendung, die selbst keine konservierenden Eigenschaften aufweisen, müssen zur Sicherstellung der mikrobiologischen Haltbarkeit während der Herstellung, der Lagerung sowie nach Anbruch im Falle der Mehrfachentnahme ausreichend konserviert werden. Mit Blick auf die pädiatrische Anwendung kommen diesbezüglich die Sorbinsäure (sowie ihre Salze), die Benzoesäure (sowie ihre Salze) und die Parabene (Methylparaben, Ethylparaben) in Frage.

Überraschenderweise hat sich nun herausgestellt, dass vor allem die Benzoesäure (sowie ihre Salze) innerhalb des erforderlichen pH-Wertes von 4,0 bis 5,0 ausreichend stabil ist und eine äußerst wirksame Konservierung der Zubereitung ermöglicht. Dabei sind Konzentrationen von 0,01 bis 0,2, bevorzugt 0,05 bis 0,15 Gew.-% und besonders bevorzugt Konzentrationen von 0,04 bis 0,12 Gew.-% wirksam. Die ausreichende Konservierung und mikrobiologische Qualität der Zubereitung sind sowohl während der Langzeitlagerung der im Behältnis abfüllten Suspension als auch nach Öffnen bzw. Anbruch über die Dauer der Entnahme aus dem Behältnis gewährleistet. Die hohe Effektivität von Benzoesäure (sowie ihrer Salze) wird auch durch den eingestellten pH-Wert von 4,0 bis 5,0 begünstigt.

Die erfindungsgemäße wässrige Suspension für die orale Anwendung enthält den arzneilich wirksamen Bestandteil Budesonid oder dessen Salze in einer Menge von 0,01 bis 0,10 Gew.-%, bevorzugt 0,010 bis 0,057 Gew.-% (entsprechend 0,1 mg bis 0,6 mg je Milliliter der Zubereitung), besonders bevorzugt 0,014 bis 0,050 Gew.-% (entsprechend 0,15 mg bis 0,5 mg je Milliliter der Zubereitung) und ganz besonders bevorzugt 0,019 bis 0,038 Gew.-% (entsprechend 0,2 bis 0,4 mg je Milliliter der Zubereitung). Die angegebene Menge gibt die Gesamtmenge des Wirkstoffs Budesonid in der Suspension wieder. In bevorzugter Ausführungsform beinhaltet die erfindungsgemäße flüssige Zubereitung zum Einnehmen keinen weiteren pharmazeutischen Wirkstoff.

Die Zubereitung enthält Budesonid in ungelöster Form. Als disperses System muss daher sichergestellt werden, dass während der Herstellung und der Anwendung eine homogene Verteilung des Wirkstoffes in der flüssigen kohärenten Außenphase vorliegt. Die Sinkgeschwindigkeit von Budesonid ist nach dem Stokes-Gesetz direkt proportional der Partikelgröße der Feststoffteilchen und der Dichtedifferenz von fester und flüssiger Phase sowie umgekehrt proportional der Viskosität des Dispersionsmittels.

Die Erfindung enthält Budesonid in mikronisierter Form. In bevorzugter Ausführung ist die Verteilung der Partikelgröße von Budesonid zu 95% < 10 µm, in besonders bevorzugter Ausführung zu 100% < 10 µm und in ganz besonders bevorzugter Ausführung zu 100% < 10 µm, 95% < 5 µm und 80% < 3 µm. In dieser Ausführungsform lässt sich der Wirkstoff gleichmäßig in der kohärenten Außenphase der Suspension verteilen. Nach Redispergierung durch Aufschütteln liegt für mindestens 2 Minuten eine homogene Wirkstoffverteilung vor.

Es tritt während dieses Zeitraumes keine Phasentrennung ein, so dass die Dosierungsgenauigkeit während der Entnahme der Suspension aus dem Behältnis sowie der anschließenden Anwendung sichergestellt ist. Weiterhin konnte gezeigt werden, dass während der Lagerung der Suspension weder Partikelgrößenwachstum noch Umlösungsvorgänge von Budesonid eintreten, die die Sedimentationseigenschaften von Budesonid ändern bzw. negativ beeinflussen können. Dies wird nur durch die erfindungsgemäße Zusammensetzung der Außenphase gewährleistet. Diese ist inert und ermöglicht die physikalische und chemische Langzeitstabilität der Suspension. Auf diese Weise kann auch auf den Zusatz von oberflächenaktiven Substanzen verzichtet werden, die in Suspensionen oft zur physikalischen Stabilisierung des dispergierten Feststoffes in der Außenphase verwendet werden. Die Partikelgrößenverteilung des Wirkstoffes wird bevorzugt durch vergleichende Lichtmikroskopie bestimmt. Dabei wird die Teilchengröße von in der Suspension dispergiertem Budesonid direkt mit der Teilchengröße des reinen Wirkstoffes verglichen.

Der Gegenstand der Erfindung enthält auch Rohrzucker (Saccharose) in einer Menge von 5 bis 25 Gew.-% zur Angleichung der Dichte zwischen der Innen- und der Außenphase der Suspension. Eine Reduktion der Dichtedifferenz wird bevorzugt durch eine Menge von 5 Gew.-%, besonders bevorzugt durch eine Menge von 7,5 Gew.-% und ganz besonders bevorzugt durch eine Menge von 10 Gew.-% erzielt. Natürlicher Rohrzucker eignet sich besonders gut als Süßungsmittel für die pädiatrische Anwendung, da auf diese Weise auf die weniger günstigen und meist synthetischen Süßstoffe verzichtet werden kann. Die Dichte der Suspension wird bevorzugt nach der European Pharmacopea gemäß der Monographie 2.2.5 bestimmt.

Das für die Behandlung von Entzündungen der Speiseröhre erforderliche Fließverhalten der Suspension wird durch den Zusatz eines indifferenten kolloidalen Hilfsstoffes erreicht. Viskositätserhöhende Zusätze ermöglichen, dass die Zubereitung nach dem Schlucken langsam der Schleimhaut entlanggleitet und den Wirkstoff auf diese Weise gleichmäßig verteilt. Bioadhäsive Eigenschaften des kolloidalen Hilfsstoffes verlängern die Verweildauer der Suspension an der Schleimhaut zusätzlich und verlängern die Transitzeit, bis der Wirkstoff in den Magen gelangt.

Als ein in dieser Hinsicht besonders geeigneter Hilfsstoff hat sich überraschenderweise Methylcellulose herausgestellt, die bevorzugt in Konzentrationen von 1.0 bis 5,0 Gew.-% und besonders bevorzugt in Konzentrationen von 1,5 bis 2,0 Gew.-% eingesetzt wird. Dabei wird eine Viskosität erhalten, die zwischen 500 und 2000 mPa*s, bevorzugt 825 mPa*s und 1540 mPa*s liegt. Die Viskosität der Suspension wird als dynamische Viskosität und bevorzugt nach der European Pharmacopea gemäß der Monographie 2.2.10 bestimmt. Die Angabe der Viskosität erfolgt entweder in Pascalsekunden bzw. Millipascalsekunden (mPa*s) oder in N*s*m⁻². Die so eingestellte Viskosität der erfindungsgemäßen Suspension ermöglicht zum einen eine ausreichend lange und homogene Verteilung des Wirkstoffes in der Außenphase nach dem Aufschütteln aber auch ein langsames Entlanggleiten der Suspension auf der Schleimhaut der Speiseröhre nach Verabreichung. Durch die bioadhäsiven Eigenschaften von Methylcellulose wird zudem sichergestellt, dass der Wirkstoff am Zielort haften bleibt.

Eine weitere, bevorzugte Hilfsstoffkomponente, die die Akzeptanz der Zubereitung für die pädiatrische Anwendung erhöht, ist künstliches Aroma, wobei Orangen- bzw. Cassisaroma besonders bevorzugt ist. Das Orangenaroma wird dabei bevorzugt in einer Menge von 0,05 bis 0,30 Gew.-%, das Cassisaroma in einer Menge von 0,015 bis 0,15 Gew.-% eingesetzt. Der kombinierte Zusatz des Aromas sowie des Süßungsmittels und die Verwendung der Zitronensäure hat sich als besonders geeignet erwiesen, den bitteren Geschmack des Budesonids zu überdecken.

Die angegebenen Gewichtsprozent beziehen sich auf das Gewicht der jeweiligen Komponente bezogen auf die fertige Suspension. Es ist selbstverständlich, dass alle Komponenten der wässrigen Suspension sich auf 100 Gew.-% addieren müssen. Wenn eine Komponente in höherer Konzentration eingesetzt wird, muss natürlich eine andere Komponente reduziert werden. Die Auffüllung der wässrigen Suspension auf 100 Gew.-% erfolgt regelmäßig durch Zugabe von für die pharmazeutische Verabreichung geeignetem gereinigtem Wasser.

Die erfindungsgemäße flüssige Zubereitung kann aus einem Mehrdosenbehältnis wie auch aus einem Einzeldosisbehältnis verabreicht werden. Die Größe des Mehrdosenbehältnisses erlaubt die Mehrfachentnahme der Suspension über eine Behandlungsdauer von mindestens 14 Tagen. Bevorzugt werden 2,5 ml bis 5 ml der flüssigen Zubereitung mit Hilfe einer geeigneten Dosierhilfe aus dem Behältnis entnommen. Diese Menge entspricht einer Dosis. Dieses Volumen kann von Kindern problemlos als ein Schluck eingenommen werden. Die Suspension wird dadurch gleichmäßig auf der Schleimhaut der Speiseröhre verteilt.

Als Dosierhilfe zur Entnahme und Anwendung eignet sich ein geeigneter Meßlöffel oder bevorzugt eine Kunststoffdosierspritze aus Polypropylen und Polyethylen niederer Dichte von 5 Millilitern, die zur Entnahme in den Adapter, der sich im Flaschenhals befindet, gesteckt wird. Die Dosierspritze besteht aus Gehäuse und Kolben und trägt auf dem Gehäuse eine Skalierung in 0,5-Milliliter-Einheiten. Zur sicheren Aufbewahrung sind die Behältnisse mit einem kindergesicherten Schraubverschluss mit Originalitätsring dicht verschlossen. Der Schraubverschluss enthält den Adapter für die Dosierspritze, der nach dem ersten Öffnen der Kappe im Flaschenhals verbleibt. Da die erfindungsgemäße Suspension in unterschiedlichen Wirkstoffkonzentrationen hergestellt werden kann, und das Volumen einer Dosis von 2,5 bis 5 Millilitern ebenfalls variabel ist, kann auf diese Weise jede erforderliche Dosis eingestellt und verabreicht werden. Diese Flexibilität in der Dosierung ist in der Pädiatrie besonders wichtig. Neben der Dosierspritze können auch andere Dosierhilfen, wie zum Beispiel geeignete Messbecher oder Dosierlöffel, zur Entnahme und Anwendung verwendet werden.

Als Behältnis werden üblicherweise Braunglasflaschen oder Kunststofflaschen aus Polyethylenterephthalat verwendet. Für eine Behandlungsdauer von mindestens 14 Tagen eignen sich besonders Behältnisse mit einem Volumen von 200 Milliliter. Die Behältnisse sowie die Schraubverschlüsse und Dosierhilfen werden nur in der für pharmazeutische Anwendung erforderlichen Qualität verwendet.

Bei der Verwendung von Mehrdosenbehältnissen muss sichergestellt werden, dass die Zubereitung nach dem ersten Öffnen und als Anbruch während der Entnahmedauer die physikalisch-chemische und mikrobiologische Qualität beibehält. Für die erfindungsgemäße Suspension konnte nun gezeigt werden, dass die Anbruchstabilität über eine Dauer von 3 Wochen gewährleistet werden kann.

Neben der Anwendung als Mehrdosenbehältnis kann die erfindungsgemäße Suspension auch in Schlauchbeutel aus Verbundfolie abgefüllt werden. In dieser Aufmachung besteht dann die Möglichkeit, die Zubereitung als Einzeldosis zu verabreichen, indem der gesamte Beutelinhalt nach Öffnen einmalig entnommen wird. Das Füllvolumen je Schlauchbeutel bzw. Stick beträgt bevorzugt 2,5 bis 5 Milliliter. Auch mit dieser Anwendung können unterschiedliche Dosisbereiche abgedeckt werden, da neben dem Füllvolumen die Konzentration der Wirkstoffsuspension variiert werden kann. Das Material der Beutel ist ein wasserdampf- und sauerstoffdichter Packstoff aus Aluminiumverbund. Die durch Heißsiegelung verschweissbaren Sperrschicht- bzw. Barrierefolien sind Alumiumverbundfolien und bestehen bevorzugt aus Polyethylenterephthalat, Aluminium und Polyethylen niederer Dichte.

Bevorzugte Ausführungsformen der vorliegenden Erfindung werden durch die nachfolgenden Beispiele verdeutlicht.

### Beispiel 1:

Überraschenderweise wurde gefunden, dass die physikalische, chemische und mikrobiologische Stabilität der Budesonid-Suspension von bis zu 24 Monaten, sowie die für die Anwendung und therapeutische Wirksamkeit der Suspension relevanten Qualitätsparameter wie Dichte, Viskosität, Redispergierung und Partikelgrößenverteilung von Budesonid durch die in Tabelle 1 angegebene qualitative und quantitative Zusammensetzung erzielt werden kann.

In Tabelle 1 sind besonders bevorzugte Mengenangaben der erfindungsgemäß bevorzugten Komponenten wiedergegeben. Die in der Tabelle wiedergegebenen Werte müssen nicht zwangsläufig völlig exakt eingehalten werden. Allerdings sind die wesentlichen Komponenten und die Verhältnisse der einzelnen Komponenten zueinander entscheidend für die vorteilhaften Eigenschaften der erfindungsgemäßen Budesonid Suspension. Für den Wirkstoff sind zwei bevorzugte Mengen angegeben. Um sicherzustellen, dass die Gesamtmenge konstant bleibt, erfolgt bei abweichender Zusammensetzung der Komponenten der Ausgleich immer durch den Hilfsstoff Wasser.

**Tabelle 1: Zusammensetzung von bevorzugten Budesonid-Suspensionen**

| **Zusammensetzung [mg/5 ml Dosis] bzw [%]** | | |
|---|---|---|
| | [mg/5 ml Dosis] | [%] |
| Budesonid | 1,0 bis 2,0¹ | 0,02 bis 0,04 |
| Natriumbenzoat | 6,2 | 0,12 |
| Ethylendiaminessigsäure, Dinatriumsalz | 5,0 | 0,10 |
| Zitronensäure, wasserfrei | 3,5 | 0,07 |
| Saccharose | 500,0 | 9,50 |
| Methylcellulose | 100,0 | 1,90 |
| Cassisaroma | 4,0 | 0,08 |
| Wasser | 4635,1 bis 4636,1 | 88,19 bis 88,21 |
| Summe: | 5255,8 | 100,00 |

| | | |
|---|---|---|
| ¹ Konzentration: 0,2 bis 0,4 mg/ml | | |

### Beispiel 2:

Die Ergebnisse der Haltbarkeitsuntersuchungen der Budesonid-Suspension bei unterschiedlichen Lagerungsbedingungen sind in Tabelle 2 zusammengefasst. Im Vergleich zu den Startwerten zeigten sich selbst bei 30°C-Lagerung keine relevanten Veränderungen. Die Tabelle zeigt die Ergebnisse der Haltbarkeitsuntersuchungen der Suspension mit einer Wirkstoffkonzentration von 0,4 mg/ml.

**Tabelle 2: Stabilitätsergebnisse der bevorzugten Budesonid-Suspension (Wirkstoffkonzentration 0,4 mg/ml)**

| | **Lagerungsdauer bei 25°C/60% relative Feuchte in Monaten** | | | | | |
|---|---|---|---|---|---|---|
| | Initial | 3 | 6 | 9 | 12 | 18 |
| pH-Wert | 4,3 | 4,3 | 4,3 | 4,3 | 4,3 | 4,2 |
| Dichte [g/ml] | 1,043 | 1,041 | 1,042 | 1,044 | 1,044 | 1,044 |
| Viskosität [mPa*s] | 1116 | 958 | 954 | 1030 | 960 | 861 |
| Redispersibilität [%]¹ | 95,9 | 101,6 | 100,5 | 99,7 | 99,0 | 99,0 |
| Partikelgrößenverteillung des Wirkstoffes [Mikroskopie] ² | Entspricht, da unverändert | Entspricht, da unverändert | Entspricht, da unverändert | Entspricht, da unverändert | Entspricht, da unverändert | Entspricht, da unverändert |
| Gehalt Budesonid [%] | 96,5 | 96,5 | 95,5 | 97,5 | 96,5 | 96,0 |
| Summe an Gesamtverunreinigungen [%] | 0,0 | 0,0 | 0,0 | 0,10 | 0,0 | 0,28 |
| Mikrobiologische Qualität³ | | | | | | |
| Gesamtkeimzahl [KBE/g] | < 10 | Nicht bestimmt | Nicht bestimmt | Nicht bestimmt | < 10 | Nicht bestimmt |
| Pilze [KBE/g] | < 100 | | | | < 100 | |
| *Escherichia coli* | Abwesend | | | | Abwesend | |

| | **Lagerungsdauer bei 30°C/60% relative Feuchte in Monaten** | | | | | |
|---|---|---|---|---|---|---|
| | Initial | 3 | 6 | 9 | 12 | |
| pH-Wert | 4,3 | 4,3 | 4,3 | 4,3 | 4,2 | |
| Dichte [g/ml] | 1,043 | 1,038 | 1,043 | 1,043 | 1,043 | |
| Viskosität [mPa*s] | 1116 | 1034 | 908 | 1191 | 917 | |
| Redispersibilität [%]¹ | 95,9 | 97,5 | 95,9 | 97,5 | 96,5 | |
| Partikelgrößenverteil lung des Wirkstoffes [Mikroskopie]² | Entspricht, da unverändert | Entspricht, da unverändert | Entspricht, da unverändert | Entspricht, da unverändert | Entspricht, da unverändert | |
| Gehalt Budesonid [%] | 96,5 | 96,5 | 95,5 | 97,5 | 96,5 | |
| Summe an Gesamt verunreinigungen [%] | 0,0 | 0,0 | 0,0 | 0,10 | 0,0 | |

| | | | | | | |
|---|---|---|---|---|---|---|
| ¹ Die Redispersibilität wird 2 Minuten nach Aufschütteln der Suspension bestimmt. Dazu wird die Budesonid-Konzentration mit HPLC bestimmt. ² Die Partikelgrößenverteilung des Wirkstoffes wird bevorzugt durch vergleichende Lichtmikroskopie bestimmt. Dabei wird die Teilchengröße von in der Suspension dispergiertem Budesonid direkt mit der Teilchengröße des reinen Wirkstoffes verglichen. ³ Die mikrobiologische Qualität der Budesonid-Suspension wird bevorzugt nach der European Pharmacopoea der Monographie 5.1.4.-1 bestimmt. ¹ Die Redispersibilität wird 2 Minuten nach Aufschütteln der Suspension bestimmt. Dazu wird die Budesonid-Konzentration mit HPLC bestimmt. ² Die Partikelgrößenverteilung des Wirkstoffes wird bevorzugt durch vergleichende Lichtmikroskopie bestimmt. Dabei wird die Teilchengröße von in der Suspension dispergiertem Budesonid direkt mit der Teilchengröße des reinen Wirkstoffes verglichen. | | | | | | |

### Beispiel 3:

Als wasserhaltige Zubereitung muss die Budesonid-Suspension konserviert werden. Wie in **Tabelle 3** gezeigt, lässt sich eine ausreichende Konservierung bevorzugt mit Benzoesäure (sowie ihrer Salze) erzielen. Dieses Konservierungsmittel kann auch bei Langzeitlagerung in der erfindungsgemäßen Zusammensetzung stabil gehalten werden. Wie die Ergebnisse von Haltbarkeitsuntersuchungen eindrucksvoll offenlegen, werden andere Konservierungsmittel, wie zum Beispiel die Sorbinsäure (sowie ihre Salze), deutlich schneller abgebaut und sind somit weniger geeignet, die mikrobiologische Stabilität der Budesonid-Suspension bei Langzeitlagerung sowie nach Anbruch des Behältnisses im Falle der Mehrfachentnahme zu gewährleisten.

**Tabelle 3: Stabilitätsergebnisse von Budesonid-Suspensionen mit unterschiedlichen Konservierungsmitteln (Wirkstoffkonzentration 0,4 mg/ml; Konservierungsmittel: Kaliumsorbat oder Natriumbenzoat)**

| | **Lagerungsdauer bei 25°C/60% relative Feuchte in Monaten** | | | | |
|---|---|---|---|---|---|
| | Initial | 3 | 6 | 9 | 12 |

| Budesonid-Suspension mit 0,1 Gew.-% Kaliumsorbat als Konservierungsmittel | | | | | |
|---|---|---|---|---|---|
| Gehalt Kaliumsorbat [%] | 103 | 93 | 95 | 79 | 83 |

| Budesonid-Suspension mit 0,1 Gew.-% Natriumbenzoat als Konservierungsmittel | | | | | |
|---|---|---|---|---|---|
| Gehalt Natriumbenzoat [%] | 97 | 106 | 100 | 102 | 95 |
| | | | | | |

| | **Lagerungsdauer bei 30°C/60% relative Feuchte in Monaten** | | | | |
|---|---|---|---|---|---|
| | Initial | 3 | 6 | 9 | 12 |

| Budesonid-Suspension mit 0,1 Gew.-% Kaliumsorbat als Konservierungsmittel | | | | | |
|---|---|---|---|---|---|
| Gehalt Kaliumsorbat [%] | 103 | 92 | 94 | 88 | 67 |

| Budesonid-Suspension mit 0,1 Gew.-% Natriumbenzoat als Konservierungsmittel | | | | | |
|---|---|---|---|---|---|
| Gehalt Natriumbenzoat [%] | 97 | 102 | 101 | 103 | 96 |

### Beispiel 4:

Die erfindungsgemäße Zusammensetzung zeigt auch nach Öffnen und der Entnahme der Suspension über eine Dauer von 3 Wochen keine Veränderungen. Die Budesonid-Suspension mit der bevorzugten qualitativen und quantitativen Zusammensetzung bleibt auch während des Gebrauchs stabil. Für die Bestimmung der Anbruchstabilität wurde die Suspension über eine Dauer von 2 Wochen aus dem Behältnis entnommen und anschließend nochmals für eine weitere Woche stehengelassen. Wie die Ergebnisse in **Tabelle** 4 eindrucksvoll zeigen, ist die Stabilität der Budesonid-Suspension im Anbruch gewährleistet.

**Tabelle 4: Anbruchstabilität der bevorzugten Budesonid-Suspension (Wirkstoffkonzentration 0,4 mg/ml)**

| | **Lagerungsdauer bei Umgebungsbedingungen in Wochen** | |
|---|---|---|
| | Initial | 3 |
| pH-Wert | 4,3 | 4,3 |
| Gehalt Budesonid [%] | 99,5 | 95,0 |
| Summe an Gesamtverunreinigungen [%] | 0,30 | 0,16 |
| Mikrobiologische Qualität¹ | | |
| Gesamtkeimzahl [KBE/g] | < 10 | < 10 |
| Pilze [KBE/g] | < 100 | < 100 |
| *Escherichia coli* | Abwesend | Abwesend |

| | | |
|---|---|---|
| ¹ Die mikrobiologische Qualität der Budesonid-Suspension wird bevorzugt nach der European Pharmacopoea der Monographie 5.1.4.-1 bestimmt. | | |

### Beispiel 5:

### Klinische Daten:

In einer 4-armigen doppel-blinden, randomisierten, placebo-kontrollierten Multicenter-Studie der Phase-II wurden einer 2x2mg/Tag oralen viskosen Budesonid-Suspension (BUU) oder Placebo bei der Behandlung von aktiver eosinophiler Ösophagitis verglichen. Die Verblindung wurde durch die Anwendung der "Double-Dummy"-Technik gewährleistet. Ziel der Studie war es, die Überlegenheit der Budesonidformulierung gegenüber Placebo zu zeigen. Der erste primäre Teilendpunkt dieser Studie war die Rate der histologischen Remission, wobei die Eosinophilen (eos) der Patienten in Remission eine mittlere Zahl von <16eos/mm² hpf ("high power field", also das Gesichtsfeld im Mikroskop bei einer 400x Vergrößerung) nach 2 Wochen Behandlung erreichen mussten. Als zweiter primärer Teilendpunkt wurde die Differenz in der mittleren Zahl der eos/mm² hpf zwischen Studienstart und dem Ende der Behandlung gemessen. Beide beschriebenen Wirksamkeitsparameter wurden konfirmativ in einem geschlossenen Testverfahren überprüft, damit ein Vergleich aller Verumgruppen mit der Placebogruppe möglich wurde.

Das Design dieser Studie einschließlich der beschriebenen Endpunkte ist nahezu identisch zur Studie, wie sie in der Publikation von Straumann, 2010 aaO beschrieben wurde.

Überraschenderweise waren die Ergebnisse der Testformulierungen nicht nur signifikant besser als Placebo, sondern auch besser als die Budesonidformulierung, welche in der Arbeit von Straumann et al., 2010 aaO beschrieben ist.

Tabelle 5 zeigt die Ergebnisse für die histologische Remission, definiert als im Mittel <16eos/mm² hpf. Patienten, die vorzeitig die Studie beendeten, ohne dass eine histologische Folgeuntersuchung stattgefunden hat wurden als Patienten, die nicht in Remission sind, analysiert. In einer Sensitivitätsanalyse wurden nur solche Patienten analysiert, die die Studie komplettiert hatten.

**Tabelle 5: Histologische Remission**

| | **Anzahl (%) der Patienten, die eine histologische Remission aufwiesen** | | | | |
|---|---|---|---|---|---|
| | **BUU-2/EEA** | | | **Straumann (2010 aaO)** | |
| | | **Budesonid 2x2 mg Suspension** | **Placebo** | **Budesonid 2x1 mg suspension** | **Placebo** |
| **FAS** | | | | | |
| At wk 12** (LOCF) ITT | | 18/19 **(95%)** | 0/19 **(0%)** | 13/18 **(72%)** | 2/18 (11 %) |
| [95% RCI] | | [57.6%; 99.5%] | --- | NA | --- |
| p-value * | | < **0.0001** | --- | < **0.0001** | --- |

| **PP** | | | | | |
|---|---|---|---|---|---|
| At wk 12** (LOCF) ITT | | 17/17 **(100%)** | 0/17 **(0%)** | --- | --- |
| [95% RCI] | | [61.3%; 100%] | --- | --- | --- |
| p-Wert * | | **< 0.0001** | --- | --- | --- |

| | | | | | |
|---|---|---|---|---|---|
| Histologische Remission definiert als < 5 eos/hpf (entsprechend <16 eos/mm² hpf) FAS, Full Analyse Set; PP, Per-Protokoll Analyse Set; RCI, Wiederholter Zuverlässigkeits-Intervall (für den Unterschied zwischen Verum und Placebo) *für Überlegenheit von Verum gegenüber Placebo **At wk 12, Bei 12 Wochen | | | | | |

Auch für die Analyse des co-primären Endpunktes konnte Überlegenheit gegenüber Placebo gezeigt werden (Tabelle 6). (Der Vergleich zu Straumann ist nicht möglich, da dieser Endpunkt bei Straumann nicht untersucht wurde).

**Tabelle 6: Differenz in der mittleren Zahl eos/mm² hpf (FAS)**

| | **Budesonid 2 mg Susp. (n=19)** | **Placebo (n=19)** |
|---|---|---|
| Durchschnitt (SD) | -96.665 (124.253) | -7.882 (157.939) |
| n | n = 18 | n = 19 |
| p-Wert * | **0.0041** | --- |

| | | |
|---|---|---|
| *für Überlegenheit von Verum gegenüber Placebo | | |

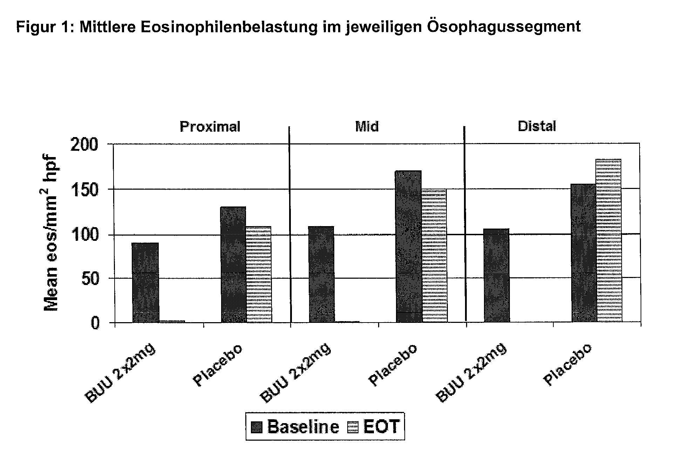

Zusätzlich konnte für alle Verumgruppen gezeigt werden, dass in allen Ösophagussegmenten die Eosinophilenbelastung nahezu komplett eliminiert werden konnte (in einen Bereich von 0,2-2,7 eos/mm² hpf). Dieses Ergebnis belegt, dass die erfindungsgemäße pharmazeutische Formulierung den Wirkstoff ösophagus-spezifisch über den gesamten Ösophagus verteilt. Dies ist in Figur 1 dargestellt.

Die Figur 1 zeigt die mittlere Eosinophilenbelastung in dem jeweiligen Ösophagussegment. Der Ösophagus wurde unterteilt in proximalen Teil, Mittelteil (Mid) und distalen Teil. Angegeben wurde die mittlere Eosinenzahl/mm² hpf. Die Abkürzung EOT bedeutet "End of Treatment".

### Beispiel 6:

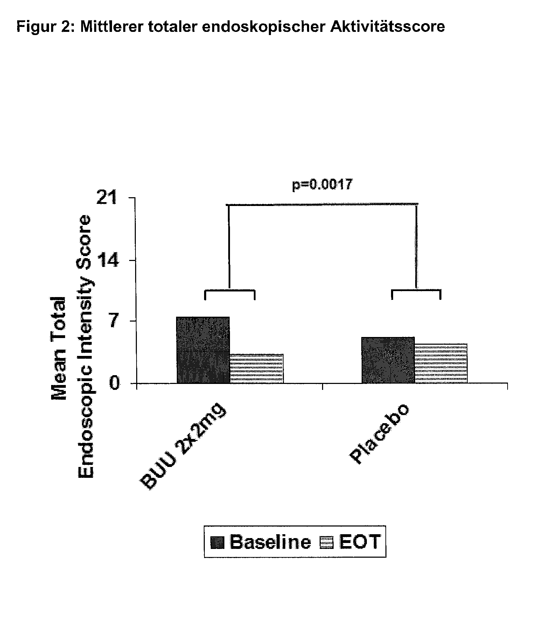

Neben der beschriebenen Wirksamkeit auf die Histologie konnte gezeigt werden, dass eine 2-wöchige Behandlung mit der erfindungsgemäßen Formulierung auch zu einer statistisch signifikanten und klinisch relevanten Verbesserung der eosinophilen Ösophagitis bezogen auf das endoskopische Bild führt. Dies ist in Figur 2 dargestellt.

In Figur 2 wird ein mittlerer endoskopischer Aktivitätsscore wiedergegeben. Die Abkürzung BUU bedeutet erfindungsgemäße, Budesonid enthaltende Suspension. Die Abkürzung 2x2 mg bedeutet die zweimalige tägliche Gabe einer Suspension mit 2 mg Wirkstoff. Baseline bedeutet die Grundlinie, EOT bedeutet "End of Treatment". Hier bedeutet Baseline die erste Visite des Patienten, an der dieser mit der Behandlung beginnt und EOT ist die letzte Visite unter der Behandlung. Da immer das Prinzip des LOCF (Last observation carried forward) angewendet wird, kann die EOT-Visite zu unterschiedlichen Zeitpunkten stattfinden, aber immer mit allen Testparametern der protokollgemässen letzten Visite des Patienten.

## Patentansprüche

1. Wässrige Suspension enthaltend 0,010 bis 0,10 Gew.-% Budesonid oder eines Salzes hiervon, **dadurch gekennzeichnet, dass** sie einen pH-Wert von 4,0 bis 5,0 aufweist.

2. Wässrige Suspension nach Anspruch 1, **dadurch gekennzeichnet, dass** sie 0,01 bis 0,5 Gew.-% eines Chelatbildners aufweist.

3. Wässrige Suspension nach Anspruch 2, **dadurch gekennzeichnet, dass** der Chelatbildner Ethylendiamintetraessigsäure oder ein Salz hiervon ist.

4. Wässrige Suspension nach einem der vorhergehenden Ansprüche, **dadurch gekennzeichnet, dass** sie 0,01 bis 0,5 Gew.-% einer pharmazeutische akzeptablen Säure oder eines Salzes davon enthält.

5. Wässrige Suspension nach Anspruch 4, **dadurch gekennzeichnet, dass** die pharmazeutisch annehmbare Säure oder das Salz davon ausgewählt ist aus Citronensäure, Weinsäure, Essigsäure, Milchsäure oder deren Mischungen sowie deren Salze.

6. Wässrige Suspension nach einem der vorhergehenden Ansprüche, **dadurch gekennzeichnet, dass** sie zusätzlich ein Konservierungsmittel enthält.

7. Wässrige Suspension nach Anspruch 6, **dadurch gekennzeichnet, dass** das Konservierungsmittel ausgewählt ist aus Sorbinsäure und deren Salzen, Benzoesäure und deren Salze und Parabene, sowie Mischungen hiervon.

8. Wässrige Suspension nach Anspruch 7, **dadurch gekennzeichnet, dass** das Konservierungsmittel Benzoesäure und ihre Salze ist.

9. Wässrige Suspension nach einem der vorhergehenden Ansprüche, **dadurch gekennzeichnet, dass** das Budesonid in mikronisierter Form vorliegt.

10. Wässrige Suspension nach Anspruch 9, **dadurch gekennzeichnet, dass** 95 % der Partikel von Budesonid einen Durchmesser von weniger als 10 µm aufweisen.

11. Wässrige Suspension nach einem der vorhergehenden Ansprüche, **dadurch gekennzeichnet, dass** sie als Süßungsmittel Saccharose in einer Menge von 1,0 bis 20 Gew.-% enthält.

12. Wässrige Suspension nach einem der vorhergehenden Ansprüche, **dadurch gekennzeichnet, dass** sie 0,5 bis 3,5 Gew.-% Methylcellulose enthält.

13. Wässrige Suspension nach einem der vorhergehenden Ansprüche zur Behandlung einer entzündlichen Erkrankung der Speiseröhre.

14. Wässrige Suspension nach Anspruch 13, **dadurch gekennzeichnet, dass** die entzündliche Erkrankung der Speiseröhre eine eosinophile Ösophagitis ist.
